(19)

Europäisches Patentamt
European Patent Office
Office européen des brevets

(11) **EP 4 443 442 A1**

(12) **EUROPEAN PATENT APPLICATION**

(43) Date of publication:
**09.10.2024 Bulletin 2024/41**

(21) Application number: **23166587.8**

(22) Date of filing: **04.04.2023**

(51) International Patent Classification (IPC):
***G16H 50/50*** *(2018.01)*

(52) Cooperative Patent Classification (CPC):
**G16H 50/50**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Charité - Universitätsmedizin Berlin
10117 Berlin (DE)**

(72) Inventors:
- **RITTER, Petra
  10115 Berlin (DE)**
- **SCHIRNER, Michael
  12247 Berlin (DE)**

(74) Representative: **Pfenning, Meinig & Partner mbB
Patent- und Rechtsanwälte
Joachimsthaler Straße 10-12
10719 Berlin (DE)**

(54) **TUNING A BIOLOGICAL NETWORK MODEL**

(57)     Computer-implemented method for tuning a biological network model, and corresponding system, based on measured data of a biological system. The method comprises obtaining the measured data of the biological system and initializing the biological network model based on the measured data. The biological network model, which comprises units, connectivity information, and weights including long range excitation weights and feed forward inhibition weights, is then tuned by repeatedly comparing a simulated synchronization of the biological network model and a measured functional synchronization of the biological system, and adapting the weights based on a deviation between said simulated synchronization of the biological network model and said measured functional synchronization of the biological system.

FIG. 5

EP 4 443 442 A1

**Description**

The present patent application is directed at a computer-implemented method for tuning a biological network model and at a computer-implemented method for determining simulated activation currents of a biological network model.

Modelling of biological systems can allow for predicting the output of a biological system based on a simulated input without needing to physically use (and possibly damage) the biological system itself. Thus, biological modelling is in particular of interest in the context of complex and fragile biological systems, which might easily be damaged when physically interfered with.

One example of a complex and fragile biological system is the human and animal body. Therefore, biological modelling of is of particular interest in the context of personalized medicine, which is directed at the customization of medical treatments to an individual patient, such as the patient's individual physiology.

For example, patients suffering from neurological disorders, e.g. degenerative neurological disorders such as Parkinson's disease may benefit from neu-rostimulation, where a stimulation electrode may be implanted into the brain. However, to maximize the positive effect on the patient, the precise location of such a stimulation electrode is crucial. Individual brain modelling, in theory, allows for predicting the effects of stimulation of a specific area of the brain on other areas of the brain, thereby allowing for a precise planning of the implantation site, thereby reducing the risk of injury to the patient.

Such simulation may, for example be based on non-invasive imaging data, for example magnetic resonance imaging (MRI), functional MRI (fMRI), diffusion weighted MRI and PET, electroencephalograms (EEG), CT scans or the like.

[Reference 1]: Deco et al: "How Local Excitation/Inhibition Ratio Impacts the Whole Brain Dynamics", in The Journal of Neuroscience 34(23), pp 7886 - 7898, discusses locally restraining feedback inhibition of the global dynamics of a large scale brain model to compensate for excess of long-range excitatory connectivity.

While methods for individual brain modelling are, in principle, already known in the literature, the prediction accuracy is still not ideal and leaves room for improvement. With an increased modelling and prediction accuracy, the planning of any surgical intervention is improved, thereby further reducing the risk to the patient while increasing the chance of success of an intervention which was planned based on the obtained model. Furthermore, more precise modelling and prediction of the activity of a biological system saves resources including expensive and limited medical resources and laboratory resources including laboratory animals.

Similar concepts also apply in other medical areas, such as cardiac stimulation or neurological stimulation outside of the brain, drug development, medical imaging, surgical simulation, disease modelling, pharmacokinetics/dynamics and/or personalised medicine.

It is therefore an objective of the present invention to provide an improved computer-implemented method for tuning biological network models based on non-invasive measured data to better estimate the effects of stimulation prior to any interference with the actual biological system, such as for example prior to any neurosurgical intervention.

This objective is addressed by the computer-implemented methods according to the independent claims. Further preferred embodiments are also described with reference to the dependent claims and enclosed figures.

One aspect of the invention is directed at a computer-implemented method for tuning an individual biological network model based on measured data of a biological system, wherein said measured data comprises structural data and activity data of the biological system.

The biological system for which a respective biological model is tuned by the computer-implemented algorithm according to this application is usually a complex biological system which is easily damaged by experiments. Thus, obtaining an accurate model by the computer-implemented tuning described herein offers great advantages as resources may be preserved and, in the medical field, damage to a patient may be reduced.

The biological system based on which the tuning is performed may for example be a mammalian brain such as a primate or human brain or a rodents brain. The biological system can, however, also alternatively relate to neurological activity data measured outside of the brain such as on the heart or on the extremities of a mammal, preferably a primate or human. Furthermore, the biological system could also relate to any other biological entity which allows for the measurement of structural activity, i.e. activity that can be measured in a structured manner, wherein activity could relate to the measurement of electrical currents, fluid levels etc. of any biological entity such as individual plants, animals or fungi or even of entire plant, animal or fungi populations, wherever a structural activity is measurable.

The method may comprise, as one step, the obtaining of the measured data of the biological system, the measured data comprising the structural data of the biological system and the activity data of the biological system.

The measured data may be pre-measured and could come from an external database. Alternatively the data may be measured specifically for the application of the disclosed computer implemented method.

The measured data may, therefore, relate to measured electrical activity, oxygen levels, for example blood oxygen levels, tissue oxygenation levels, mechanical activity, or any other measured substance concentration, such as ion concentrations as well as structural or activity information reported in the published neuroscience literature corpus or the like.

When the biological system relates to a mammalian brain, the measured data preferably refers to data which is measured non-invasively, for example using EEG or, preferably, MRI data (such as fMRI, dwMRI, etc), which provides an advantage due to the high spatial resolution.

Structural data of the measured data may refer to data relating to the locations of the respective measurement sites. For example measuring sites may be identified based on a plurality of sensors which are located to measure an activity of the respective measurements sites. Alternatively, in case of measuring via imaging data such as fMRI based measuring data, measurement sites may still be identified based on pre-defined locations within the measured structure, for example based on a pre-defined brain atlas. Structural data may also relate not only to location data but also to connection data, which shows biological connections between different locations of the biological system. For more details regarding structural data and brain atlases, please see [Reference 2]: Glasser, Matthew F., et al. "A multi-modal parcellation of human cerebral cortex." Nature 536.7615 (2016): 171-178, for details on brain atlases; and

[Reference 3]: Sporns, Olaf, Giulio Tononi, and Rolf Kötter. "The human connectome: a structural description of the human brain." PLoS computational biology 1.4 (2005): e42, for details regarding brain connections and their respective models.

Activity data of the biological system, on the other hand, relates to any measurement of a physical of chemical property of the biological system such as measurements of electric activity, fluid levels, mechanical activity, molecular concentration, for example blood oxygen levels (visualized using fMRI) etc, which indicate a current activity, energy or energy consumption. In particular, fMRI can be used to visualize blood oxygenation level dependent (BOLD) contrast, as discussed e.g. in

[Reference 4]: Raichle, Marcus E., et al. "A default mode of brain function." Proceedings of the national academy of sciences 98.2 (2001): 676-682.

In the measured data, the structural data and the activity data may optionally be measured jointly, so that a measured activity value can be associated with a structural value, such as a measurement location, in the measured data.

After the obtaining of the measured data of the biological system, the computer implemented method may comprise a step of obtaining a measured structural connectivity of the biological system, wherein the measured structural connectivity is determined based on the measured structural data.

The measured structural connectivity may be obtained from a database, for example the same database from which the measured structural data and or other measured structural data was obtained or the measured structural connectivity may be determined by the computer-implemented method of the present application.

The method furthermore also may comprise a step of obtaining a measured functional synchronization of the biological system, wherein the measured functional synchronization is determined based on the measured activity data.

The measured functional synchronization thereby preferably comprises a measure which indicates a degree of synchronicity of the activity of different measurement locations of the biological system. For example, the structural synchronization can be indicated by a matrix which comprises, for each pair of measurement locations a numeric value indicating a degree of synchronicity, i.e. how often within the measured data, there was a synchronous activity between a respective pair of locations, wherein the strength of activity and the synchronicity of the strength of activity may also be taken into account. Alternatively, the structural synchronization could also take synchronicity between more than two different locations into account, i.e. multi-bodied synchronicity could also possibly be taken into account.

The measured functional synchronization may also be obtained from a database or other source, for example the database or source from which the measured data was obtained or the measured functional synchronization may be determined by the computer-implemented method of this application.

The computer-implemented method furthermore comprises a step of initializing the biological network model based on the measured data, so that the biological network model comprises a set of units and connectivity information of said units. The set of units and the connectivity information is determined based on the measured data, in particular based on the measured structural data of the biological system.

For example, the set of units may correspond, at least partially to a set of measurement points of the biological system, based on which the measured data was obtained. Furthermore the connectivity information of the biological network model may at least partially correspond to the measured structural connectivity or structural data, which indicates biological, chemical and/or physical connections between different measurement points or areas.

Each unit of the set of units comprises an activity model, an inhibitory subunit and an excitatory subunit, wherein the inhibitory subunit of a given unit is structurally coupled to the excitatory subunit of the same unit and vice versa. The activity model defines a time-dependent simulated activity of the respective unit.

In this manner, it is possible for the activity model to take excitatory as well as inhibitory activity into account.

The biological model furthermore comprises a set of long range excitation weights, each of which represents a coupling strength from the excitatory subunit of one unit to the excitatory subunit of another unit.

Also, the biological network model comprises a set of feed forward inhibition weights, each of which represents a coupling strength from the excitatory subunit of one unit to the inhibitory subunit of another unit.

The respective weights allow for the current activity of one unit to influence the current or future activity of another unit where the influence may be either of an excitatory or inhibitory nature.

In an example, if the biological system is a mammalian brain, the units may each represent a brain area of varying size such as a cortical column, a cortical module, or a subcortical nucleus. The excitatory and inhibitory subunits may then represent excitatory or inhibitory neural populations, respectively, with each neural population including one or more neurons. The long range excitation weights and feed forward inhibition weights then represent a synaptic connection strength of long distance structural connections between the different brain areas. In particular, the long range excitation weights each represent a structural or physical connection strength, such as, for example, a synaptical connection strength, between the excitatory populations of two different brain areas, while the feed forward inhibition weights represent synaptical connection strength from an excitatory population of one brain area to the inhibitory population of another brain area. Furthermore, the excitatory and inhibitory populations of a single brain area also influence each other, which may be represented by additional connection weights between said units.

The computer-implemented method then comprises steps for tuning the biological network model by repeatedly performing the following steps until a stopping condition is met:

i) Determining a simulated synchronization of a selected subset of the set of units based on the simulated activity of said selected subset of the units. The simulated activity of each unit of the selected subset of the set of units is thereby determined based on the activity model of the respective unit.

ii) Determining a deviation between a simulated synchronization and a corresponding subset of the measured functional synchronization of the biological system;

iii) Adapting a proper, non-empty subset of the set of long range excitation weights and/or a proper non-empty subset of the set of feed forward inhibition weights based on the deviation.

In this manner, the weights of the biological network model are adapted individually so that the biological network model may represent and/or model the inter-relationships of the different areas of the biological system as accurately as possible.

In a preferred example, the adapting of the subset of long range excitation weights and/or feed forward inhibition weights based on the deviation may, in particular, comprise that, for a first unit and a second unit of the set of units, the long range excitation weight from the first unit to the second unit and the feed forward inhibition weight from the first unit to the second unit are adapted interdependently.

An interdependent adaptation of a long range excitation and corresponding feed forward inhibition weight may, therefore alter the ratio between excitation and inhibition between the two units, thereby allowing for a more accurate tuning of the inter-relationships of the respective units, which then increases the accuracy of the biological network model.

In a preferred example, the interdependent adaptation of the long range excitation weight and the feed forward inhibition weight from the first unit to the second unit may comprise increasing one of said two weights while decreasing the other of said two weights.

In this manner, it is possible to adapt the rate between inhibition and excitation between two units to better control the simulated activity of the biological network model.

For example, the determined deviation may be scaled by a fixed and/or dynamic scaling factor to determine a scaled deviation and the subset of long range excitation weights and/or the subset of feed forward inhibition weights may then be adapted based on the scaled deviation.

The scaling factor may, for example comprise a pre-determined and/or empirically optimized learning rate in order to reduce the risk of overfitting as well as to increase the probability of convergence. Such a learning rate may be changed over time for example according to an exponential decay, thereby reducing the learning rate over time in order to further increase the probability of convergence of the parameters, i.e. the weights which are adapted during the tuning.

Optionally, the simulated synchronization of the subset of the simulated units is determined based at least in part on the temporal correlation of the activities of said subset of the simulated units.

Optionally the biological network system is a mammalian brain and the biological network model is an individual brain network model. Furthermore, optionally the measured functional synchronization is a functional connectivity determined based on the measured data of the biological system.

In an example, the activity model may define a temporal dependency between a current excitatory activity of a specific unit and previous excitatory and/or inhibitory activities of connected units, which are connected to the specific simulated unit according to the structural connectivity information. The activity model may, furthermore, define a temporal dependency between a current inhibitory activity of the specific unit and previous excitatory and/or inhibitory activities of the connected units.

For example, the activity model may be based on simulated neural activity of the units, which may correspond to modelled spiking neurons as commonly used in spiking neural networks. The activity model may, thereby, determine firing rates of said spiking neurons and/or simulated currents related an input and/or output of the respective unit.

In particular, the specific unit's current excitatory activity may be determined based at least in part on a weighted sum over a set of previous simulated excitatory activities of the connected units, which are weighted by the respective long range excitation weights; and the specific unit's current inhibitory activity may be determined based at least in part on a weighted sum over a set of previous simulated excitatory activities of the connected units, which are weighted by the respective feed forward inhibition weights.

In this manner, a time-dependent coupling strength between different units can be taken into account when determining the activity of a specific unit.

Furthermore it is optionally possible that, in the biological network model, for at least one unit, the excitatory subunit and the inhibitory subunit of said unit are coupled by a local excitatory weight representing a coupling strength from the excitatory subunit to the inhibitory subunit, and a local inhibitory weight representing a coupling strength from the inhibitory subunit to the excitatory subunit.

Said local weights may be used to couple the subunits of at least one given unit, preferably of a plurality or all units, in order to keep the excitatory activity and inhibitory activity of a unit from diverging too much, thereby improving the result of tuning.

Additionally, it is possible for the computer-implemented method to further include a step of adapting the local inhibitory weight of the unit based on a difference between said unit's activity, such as the excitatory activity, and a target activity, wherein the target activity may for example be a target excitatory firing rate which can be preset to a predetermined value. For example, the target excitatory firing rate may be set to any value between 0.1 and 100 Hz, in some examples between 2 and 8 Hz. The target excitatory activity rate may optionally be based on an empirically determined activity of the measured data of the biological system.

This further improves the tuning results by keeping the activity of the units from diverging too much from the biological reality. The adaptation of the local inhibitory weight may furthermore only be performed if the difference between the excitatory activity of the unit and the target excitatory activity is greater than a preset threshold. Furthermore the target activity may be an interval or a set of numeric values.

Optionally, in an implementation, the biological network model may comprise a number N of units and the long range excitation weights are represented by a long range excitation matrix of size NxN, the feed forward inhibition weights are represented by a feed forward inhibition matrix of size NxN and/or the local inhibitory weights are represented by a local inhibitory vector of size N. For example, there can be between 2 and 10.000 units depending on the resolution of the measured data. Preferably, there are between 10 and 1.000 units. The number of units in particular may depend on a used brain atlas. In an example described below, a brain atlas with 379 regions is used, therefore resulting in N=379 units of the biological network model. More details regarding this particular brain atlas can be found in [Reference 3].

The number N may therein correspond to a number of measurement points or regions of the biological system based on which the measured data was obtained.

The application furthermore relates to a computer-implemented method for determining simulated activation currents of a biological network model.

Said method comprises at least the following steps:

a) Obtaining the biological network model.

b) Applying a simulated input current to each unit of a first subset of the units of the biological network model.

c) Determining the simulated activation currents of each unit of a second subset of the units of the biological network model.

The biological network model obtained in step a) comprises set of units, which correspond to areas of the biological system, and connectivity information of said units, each unit comprising an activity model, an inhibitory subunit and an excitatory subunit, wherein the excitatory subunit of a unit is functionally coupled to the inhibitory subunit of the same unit and vice versa, wherein the activity model defines a time-dependent simulated activity of the respective unit.

Furthermore, the biological network model comprises a set of long range excitation weights, each of which represents a coupling strength from the excitatory subunit of one unit to the inhibitory subunit of another unit; and set of feed-forward inhibition weights, each of which represents a coupling strength from the excitatory subunit of one unit to the inhibitory subunit of another unit.

In step b) the simulated input currents are applied to the respective units by inputting the simulated activation currents into the activity models of the respective units.

In step c) the simulated activation currents of a unit of the second subset of the units is determined based on the simulated input currents applied to the units of the first subset of the units in step b) and further based on the activity model of said unit of the second subset of the units. Thus, simulated activation currents of the units of the second subset of units can be determined either directly or iteratively.

Determining simulated activation currents of a biological network model can serve to estimate the effects of local stimulation on said biological network. For example in the case of deep brain stimulation, a simulation of the effect of deep brain stimulation can provide an important tool for planning of surgical intervention, thereby reducing the need for trial-and-error and reducing the risk for the patient as well as reducing the necessary resources.

Optionally the biological network model for which the simulated input currents are determined may have been obtained by the computer-implemented tuning method described above. In this case, possible implementation details regarding the biological network model are furthermore described with reference to the computer-implemented method for tuning the biological network model as described above.

Using a well-tuned biological network model has the additional effect of producing more realistic results, i.e. the determined simulated input currents are closer to the real-life currents of the biological system.

With respect to the tuning as well as simulation method described above, it is optionally possible for units to represent areas of the biological system of different sizes. For example, one unit may represent a first area of the biological system and another unit may represent a second area of the biological system with the first area being of substantially larger size than the second area. These units may then be considered to model the biological system on different scales. There may also be units that represent the same area on different scales, for example a set of small-scale units may together represent the same area of the biological system as a single large scale unit, thereby allowing for modelling of the biological system on different scales, which is also referred to as multi-scale modelling. This may have the advantageous effect of allowing for a more detailed modelling of areas that are considered of higher importance than others. Also during simulation, multi-scale modelling allows for simulation of input currents application to areas of different sizes.

To achieve such multi-scale modelling, the method for determining simulated activation currents may further comprise the following additional steps:

- obtaining a secondary biological network model comprising a set of secondary units and secondary connectivity information of said secondary units, wherein the set of secondary units corresponds to a different scale model of a specific unit of the set of units of the biological network model, each secondary unit comprising a secondary activity model; and
- determining simulated activation currents of at least some of the secondary units, wherein the simulated activation currents of the at least some secondary units are determined based on simulated input currents applied to at least some of the units and/or based on input currents applied to at least some of the secondary units.

The secondary biological network model may be a network model which corresponds to an area of the biological network which corresponds to the specific unit in the original biological network model, but on a different scale. For example, the specific unit may represent the entire Hippocampus while the secondary unit may represent hippocampal subfields or hippocampal neurons.

For the secondary units, a connectome including connection strengths may be known, for example from existing databases or literature, or they may be determined or estimated from medical imaging data, for example using the tuning method described herein. The activation currents of the secondary units can then be estimated based on scaled currents (either input or determined activation currents) of the specific unit in combination with the connectome, or connectivity information, which is known for the secondary biological network model. Said scaling allows for a different-scale estimation (for example a finer or larger scale estimation) of activation currents, which may be used as an aid in determining the precise effect of a stimulation of a specific area of the biological system on another area of the biological system.

The application furthermore also relates to a data processing apparatus which comprises means for carrying out the method steps of any one of the preceding claims.

In the following detailed description, an exemplary application of the claimed computer-implemented method and system to brain network modelling is described in detail. However, the subject matter of the present application may also be applied to other biological systems, such as modelling of non-brain neurology or cardiac tissue modelling.

FIG. 1     shows a schematic overview of a system for tuning a biological network model and for determining simulated activation currents of a biological network model;

FIG. 2     shows a schematic depiction of a brain with units and their connections;

FIG. 3     shows an exemplary functional connectivity matrix;

FIG. 4     shows a biological network model according to the present application comprising three units;

FIG. 5    shows a high-level flow chart for a tuning method according to this patent application;

FIG. 6    shows a detailed flow chart of a method implementation of step 501 of FIG. 5;

FIG. 7    shows a detailed flow chart of a method implementation of step 502 of FIG. 5;

FIG. 8A   shows a detailed flow chart of a method implementation of step 503 of FIG. 5;

FIG. 8B   shows a detailed flow chart of an alternative implementation of a method of step 503 of FIG. 5, which additionally comprises feedback inhibition control;

FIG. 9    shows a flowchart of a simulation method according to the present patent application.

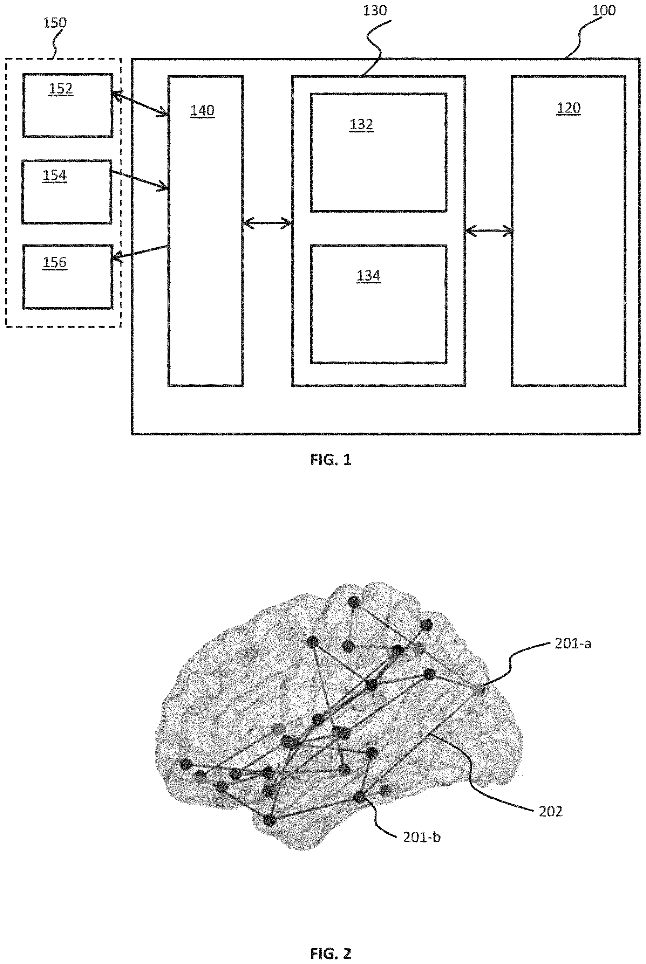

FIG. 1 shows a schematic overview of a computer-based system configured for tuning a biological network model as well as for determining simulated activation currents of a biological system based on a biological network model of said biological system. The computer-based system 100 includes a central processing unit (CPU) 120, a memory unit 130, an input/output interface 140, and several input/output devices 150 coupled to the interface 140.

The system can be a stand-alone computer or a distributed system. The CPU may include a multiprocessor and/or multi-core processor for fast algorithm and data execution. The memory unit 130 may include primary storage linked to the CPU(s), random access memory (RAM), volatile memory as well as hard disks, flash memory units etc. The memory 132 comprises modelling data 132, which is data related to the biological network model, such as measured data of the biological system, data regarding the units of the biological network model, their respective connectivity information and activity models etc. The memory further comprises instructional data 134 which comprises computer readable instructions either for tuning the biological network model based on the modelling data 132 or instructions for determining simulated activation currents based on the modelling data 132.

The input/output interface 140 can include several interfaces such as an input for a keyboard, a wired or wireless data connection for uploading or downloading data into the memory unit, or interfaces for connecting a display, such as a monitor, or a keyboard for inputting commands. As examples, the interface of Fig. 1 is coupled with the external storage 152, which may be an inter-net storage or a local external storage, a keyboard and/or mouse 154, and a display 156.

In the biological network modelling, and in particular in brain network modelling, a biological network is modelled as an interconnected network, represented by a graph, of several units, also referred to as nodes, with some of said units being connected by edges, also referred to as connections, which may be weighted to allow for representing a coupling strength between the different units.

FIG. 2 shows an image of such a brain model with exemplary measurement points, also referred to as regions or areas or units, and functional interconnections between said measurement points. Such a representation of brain regions and their interconnections is also referred to as a structural skeleton model of a brain and may be obtained based on MRI data. In the case of brain network modelling, each unit, or node, represents an area of a human brain, wherein an area may refer to a single neuron, multiple neurons (also referred to as a population of neurons) up to an entire hemisphere of the modelled brain.

A structural skeleton model may be based on a brain atlas, which may be imported from respective databases, see [Reference 2, Reference 3]. Such a brain atlas usually includes pre-defined brain regions and may also include connectivity information between the different brain regions, thereby indicating if certain brain regions are functionally connected and, possibly, a weight indicating a strength of the respective connection.

In FIG. 2, measurement points 201-a and 201-b are connected by connection 202, implying that there is some sort of functional coupling between these two measurement points or their respective brain regions. The connection can be weighted to allow for modelling a strength of connection. Note that connection strengths between brain areas are not necessarily symmetric, i.e. it is possible that the connection strength from a first brain region to a second brain region differs from the connection strength from the first brain region to the second brain region.

Connectivity information of a brain can, for example, be obtained from dwMRI data via fiber tractography, see [Reference 3]. For other biological systems, the connectivity data may similarly be obtained using respective appropriate imaging techniques.

The measurement data on which the disclosed tuning method is based, therefore comprises respective structural data, indicating the measurement points and their connections of the biological system. Additionally the measurement data also contains activity data, which provides an insight into the correlation of activity of different measurement points.

In the case of a human brain, the activity data may be obtained from fMRI data and a resulting functional connectivity matrix.

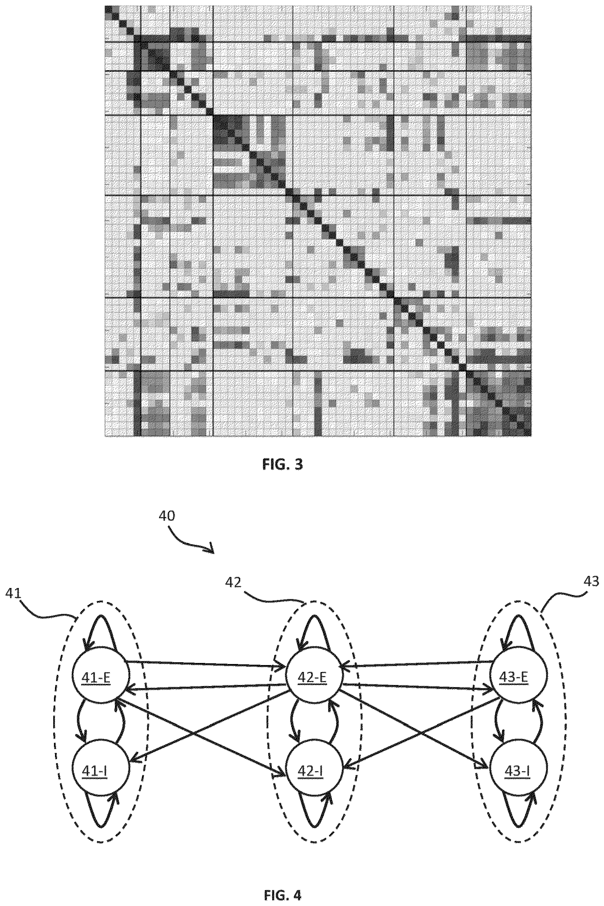

FIG. 3 shows an example of a visualization of such a functional connectivity matrix which visualizes the correlation strength between the activities of different measurement points of brain areas. Functional connectivity matrices are usually symmetric, as only temporal correlation of activity is visualized.

The functional connectivity matrix of a brain may, for example, be obtained based on the following method, which can for example be implemented based on the methods provided by fmrirep.org. The method for computing a functional connectivity matrix comprises the following basic steps.

| Step 1: | Recorded fMRI blood-oxygen-level-dependent imaging (BOLD) data is corrected for imaging artifacts, aligned with structural MRI data, and divided into a set of regions according to a given brain atlas. |
| Step 2: | The average time course of fMRI BOLD activity is computed for every atlas region. |
| Step 3: | The cross-correlation between average atlas region time courses is computed for every pair of regions i and j and the resulting values entered in the Functional Connectivity Matrix at the cells (i,j) and (j,i). |

For the example of brain network modelling, the measurement data comprises a structural representation of the respective brain in the form of a set of measurement points and their respective connection information and the measurement data furthermore comprises the functional synchronization between the measurement points in the form of a resting state functional connectivity of the measurement points, which is this represented by a functional connectivity matrix of numeric values.

The brain network model, which is to be tuned by the computer-implemented method is first initialized. During initialization, for each of a selected subset of measurement point of the measurement data, a unit of the brain network model is initialized and connectivity information is also either obtained from the measured data or taken from an imported brain atlas or pre-defined initial model.

In order to properly model a brain or other complex biological systems, such as neural system or the like, for each unit two different kinds of activities are taken into account, namely an excitatory activity as well as an inhibitory activity of the unit. While these two activity levels cannot be measured directly, research has shown that simulation results are improved when each node or unit is decomposed into an excitatory subunit and an inhibitory subunit. This concept of excitation and inhibition is, for example, also studied in the field of human intelligence and reasoning, where inhibitory activity keeps a person from acting too quickly based on their instincts, thereby allowing for more complex reasoning before a decision is taken. Inhibitory activity can, therefore, be considered as potentially important for deep thinking and for making well-planned decisions. However, even though the concept of excitation and inhibition stems from neurological research, said concept can also be successfully transferred to other biological systems, which may have a similar concept of competing activities which try to either express or suppress an activity, where an activity can be measured, for example in the form of a measurable energy burst, i.e. in the form of electric energy, or corresponding energy consumption, can also be measured by way of oxygen levels, ion levels or the like.

FIG. 4 shows a schematic depiction of a brain network model 40 comprising three exemplary units 41, 42, 43 of the brain network model 40, as discussed herein. For each unit, an excitatory subunit 41-E, 42-E, 43-E is defined as well as an inhibitory subunit 41-I, 42-I, 43-I. For each unit, the excitatory and inhibitory subunits are coupled and may, therefore, influence each other. Each unit 41, 42, 43 also is initialized with an activity model, which may be based on the known concept of spiking neurons, as disclosed in [Reference 1].

Furthermore, in the brain network model weighted connections between the units are added. In particular, the excitatory subunits of different units may be connected by long range excitation (LRE) connections, which are weighted by individual long range excitation weights. FIG. 4 shows such LRE connections between units 41 and 42 as well as between units 42 and 43. LRE connections may also exist between units 41 and 43 but are not shown in FIG. 4 for ease of notation only.

The brain network model furthermore comprises individual weighted connections between an excitatory subunit of one unit and an inhibitory subunit of another unit. These connections are referred to as feed forward inhibition (FFI) connections, which may be weighted by respective FFI weights. In FIG. 4, FFI connections are shown from the excitatory subunit 41-E to the inhibitory subunit 42-I, from the excitatory subunit 42-E to the inhibitory subunit 41-I, from the excitatory subunit 42-E to the inhibitory subunit 43-I, and from the excitatory subunit 43-E to the inhibitory subunit 42-I. Furthermore, FFI connections may also exist from the excitatory subunit 41-E to the inhibitory subunit 43-I and from the excitatory subunit 43-E to the inhibitory subunit 41-I, but these are not shown in FIG. 4 for ease of notation only.

The LRE weights and FFI weights are individually tunable in the disclosed computer-implemented tuning method, where the adaptation of an LRE weight and corresponding FFI weight (such as, e.g. the LRE weight from subunit 41-E to 42-E and the FFI weight from subunit 41-E to 42-I) are preferably adapted interdependently, thereby allowing for an adaptation of an excitation/inhibition ratio between individual units, which provides particularly good tuning results.

To keep the activities of an excitatory and corresponding inhibitory subunit (such as, for example subunits 41-E and 41-I) from diverging too much, it is additionally possible to tune a local inhibitory weight, which is a weight that influences the connection from an inhibitory subunit to the excitatory subunit of the same unit (for example from subunit 41-I to subunit 41-E). Tuning of local inhibitory weights is usually done based on a target activity of the unit, with the respective local inhibitory weight being adapted whenever the simulated activity of the unit diverges more than a predefined threshold from the predefined target activity.

The selected subset of measurement points on which the initialization is based may be pre-determined based on the application. The subset may comprise anywhere between two and all measurement points of the measured data.

Also, it is possible that during initialization of the brain network model that some measurement points are grouped together into a single unit of the brain network model or that multiple scales of measurement data are taken into account, wherein some measurement points are modelled individually while other measurement points are grouped together, or some measurement points may be modelled both individually and as a group, thereby causing multi-scale modelling of the biological system.

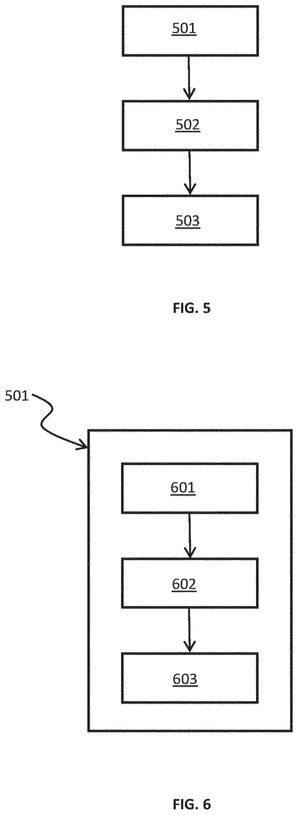

FIG. 5 shows a flowchart of the described computer-implemented method for tuning a biological network model of a biological system. The method comprises the following three basic steps, which are then further discussed in more detail below with reference to FIGs. 6, 7, 8A and 8B.

Step 501: Obtaining measured data of the biological system including measured structural connectivity information and measured functional synchronization of the biological system.

The measured data can pre-measured and be obtained from a database or can be measured specifically for the present tuning application. Some preprocessing of the measured data may be necessary in order to obtain the measured structural connectivity information and measured functional synchronization information of the biological system. The measured structural connectivity information describes the structure of the biological system and the connectedness of different areas of the biological system, while the measured functional synchronization describes how activity of different areas of the biological system is correlated.

Step 502: Initializing the biological network model based on the measured data.

In Step 502, parameters of the biological network model, such as the size of the biological network model, connections within the biological network model and functions and parameters defining the activity of the biological network model are initialized. Some of the initialization parameters may be taken from a database while other parameters are initialized based on the measured data.

Step 503: Tuning the biological network model by repeatedly performing optimization steps until a stopping condition is met.

In Step 503, parameters of the biological network model are repeatedly adapted based on an error metric determined between values computed for the biological network model and values computed for the measured data, thereby optimizing the biological network model to mimic the behaviour of the biological system.

FIG. 6 depicts a flow chart which provides further details of Step 501, i.e. the obtaining of the measured data.

Step 601: Obtaining the measured data of the biological system, the measured data comprising structural data of the biological system and activity data of the biological system.

Measured data of the biological system can be obtained based on electrical or magnetic measurements, for example based on sensors, or based on visual recordings and the like. In case of brain network modelling, measurement data usually refers to different types of MRI data, such as fMRI data, structural MRI data, dwMRI data, or to EEG data.

Step 602: Obtaining a measured structural connectivity of the biological system, wherein the measured structural connectivity is determined based on the structural data of the biological system.

Within the measured data, structural data may be either included or structural data may be determined from the measured data by predetermined data processing steps. Structural data refers to a number of regions or areas of the biological system, wherein for each such area, measured data pf the area is included in the measured data. The measured structural data furthermore comprises connectivity data, which specifies structural connections between regions, with said structural connections also being either directly included in the measured data or being determined from the measured data. Step 603: Obtaining a measured functional synchronization of the biological system, wherein the measured functional synchronization is determined based on the measured activity data.

Activity data can be measured based on either electrical data (for example when brain activity is measured using EEG), based on MRI data or based on mechanical activity (movement), concentration of ions, proteins etc.

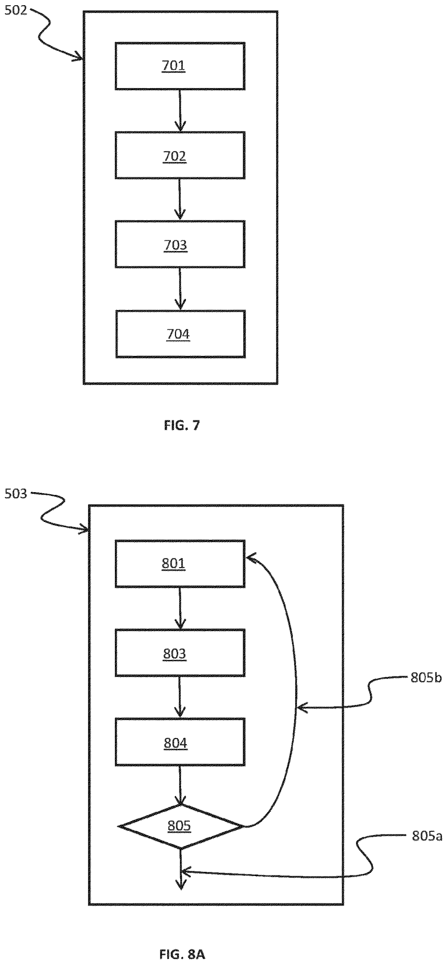

FIG. 7 depicts a flow chart which provides further details regarding Step 502, i.e. the initialization of the biological network model.

Step 701: Initializing units of the brain network based on the measured structural data.

In particular, a number of units is selected based on the number of areas of the biological system which are included in the measured structural data. It is alternatively also possible to only select, either automatically or manually, a subset of the areas of the biological system for which measurement data is available or to group some areas into area groups when initializing the corresponding units of the brain network model. For each unit, furthermore an activity model is defined and initialized activity model, which may be selected from a database or pre-defined based on the type of the biological system. The activity model defines a time-dependent simulated activity of the respective unit, for example based on model equations of spiking neurons. Each unit furthermore comprises an inhibitory subunit and an excitatory subunit, wherein the excitatory subunit of at least some units is functionally coupled to the inhibitory subunit of the same unit and vice versa, thereby allowing for the excitatory and inhibitory subunits to influence each other.

Step 702: Initializing the connectivity information of the brain network model based on the measured structural data.

The connectivity information of the units is then also determined based on measured connections between the areas of the biological system. For example, the connectivity information may be based on a known or predefined brain atlas, or the connectivity information may be determined based on dwMRI data.

Step 703: Initializing parameters of the brain network model. The brain network model may comprise predefined parameters which need to be initialized with fixed values that remain unchanged during the tuning of the brain network model. These parameters may be initialized based on a database or based on empirical knowledge or biological data, for example to mimic certain known properties of the biological system.

Step 704: Initializing tuning variables.

The biological network model comprises variables that are intended to be tuned, i.e. adapted, during the tuning process. In particular, the tuning variables comprise the set of long range excitation weights, each of which represents a coupling strength from the excitatory subunit of one unit to the excitatory subunit of another unit; and the set of feed forward inhibition weights, each of which represents a coupling strength from the excitatory subunit of one unit to the inhibitory subunit of another unit. These tuning variables are also initialized with predefined values, based on which the tuning method is then further executed. Depending on the biological network model and the underlying biological system, it may matter which initial values are chosen for the tuning variables. For example, initial tuning variable values that are too far from the optimal values of the tuning variables may cause a suboptimal tuning result.

FIG. 8A depicts more details regarding method step 503, i.e. the tuning of the initialized biological network model.

Step 801: Determining a simulated synchronization of a selected subset of the units based on the simulated activity of said selected subset of the units, wherein the simulated activity of each unit is determined based on the activity model of said unit.

For each unit, the activity model corresponds to a function or method, which determines a simulated activity based on previous activities of connected units and their connection weights. The simulated activity of a unit usually corresponds to the simulated activity of the excitatory subunit of said unit, with the simulated activity of the inhibitory subunit only being indirectly taken into account as an input to the excitatory subunit. Once the simulated activities of the selected units are determined, a pair-wise correlation is determined for each pair of selected units, which then corresponds to the determined simulated synchronization. In the case of brain network modelling, the simulated activities may correspond to firing rates of spiking neurons or to simulated input currents.

Step 803: Determining a deviation between the simulated synchronization and a corresponding subset of the measured functional synchronization of the biological system.

A deviation can, for example, be determined based on a calculated difference or distance between the simulated synchronization and the measured functional synchronization.

Step 804: Adapting a proper, non-empty subset of the long range excitation weights and/or a proper non empty subset of the feed forward inhibition weights based on the deviation.

Based on the deviation, a correction value is determined for at least some of the tunable weights and said correction value is added to the respective weights.

Step 805: Determining whether or not a stopping condition is met.

A stopping condition may be defined based on a deviation threshold, i.e. the stopping condition is considered to be met when the deviation determined in Step 803 is below a predefined threshold. Alternatively, a stopping condition may be met automatically after a pre-defined number of iterations or a predefined tuning time has passed. Alternatively, a stopping condition may be based on the value change during the adapting of Step 804, i.e. a stopping condition is met if the numeric values which are added to or subtracted from the respective weights are below a certain threshold. If the stopping condition is met, then the tuning is stopped 805a, or if the stopping condition is not yet met, then the tuning method of steps 801 to 804 is repeated.

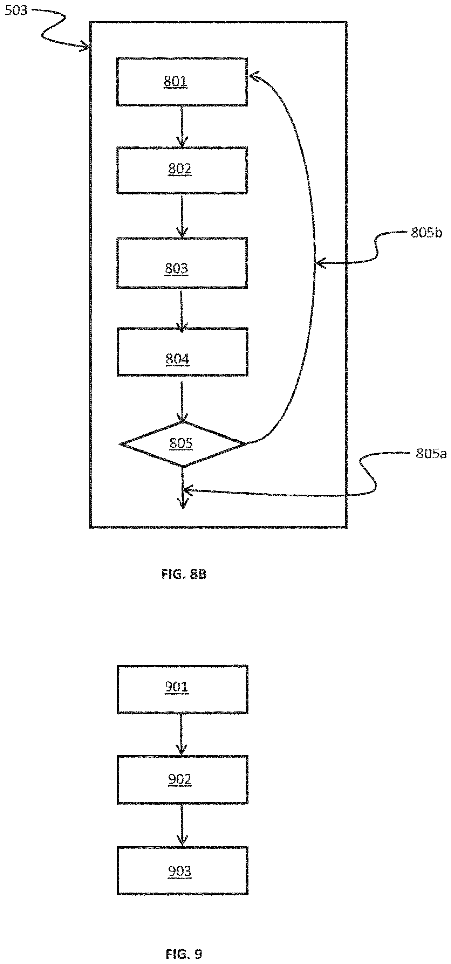

FIG. 8B depicts an alternative embodiment of a detailed implementation of Step 503, which, additionally to the method steps already shown in FIG. 8A, also includes a method step 802, which is preferably performed between steps 801 and 803 discussed above.

Step 802: Adapting a local inhibitory weight of at least one unit of the set of units if a difference between said unit's activity and a target activity exceeds a threshold.

The adaptation of step 802 serves to restrict the activities, such as the firing rates, of the units to a plausible level by way of adapting the weight between the inhibitory and excitatory subunits. The local inhibitory weights can be adapted individually for each unit.

In case of the embodiment shown in FIG 8B, the local inhibitory weights are additionally initialized in Step 704.

FIG. 9 depicts the steps of a method for determining simulated activation currents of a biological network model in accordance with an embodiment of this patent application. The method for determining simulated activation currents comprises the following steps.

Step 901: Obtaining the biological network model.

The biological network model is pre-tuned and comprises a set of units and connectivity information of the units. The units and connectivity information describe a network of different areas of the biological system as well as the interconnections between the areas of the biological system.

Each unit comprises an activity model as well as an inhibitory subunit and an excitatory subunit. The excitatory and inhibitory subunits are functionally coupled to each other and the activity model defines a time-dependent simulated activity of the respective unit.

The biological network model furthermore comprises a set of long range excitation weights, each of which represents a coupling strength from the excitatory subunit of one unit to the excitatory subunit of another unit, and a set of feed forward inhibition weights, each of which represents a coupling strength from the excitatory subunit of one unit to the inhibitory subunit of another unit.

Step 902: Applying a simulated input current to each unit of a first subset of the units.

The simulated input current allows for simulating an energy application, or stimulation, in the respective area of the biological system. The first subset of units may comprise only one unit or a plurality of units. When a plurality of units is used, different simulated input currents can be applied to the individual units of the first subset of units.

Step 903: Determining simulated activation currents of each unit of a second subset of the units, wherein the simulated activation currents of a unit of the second subset of the units is determined based on the simulated input currents applied to the units of the first subset of the units, and further based on the activity model of said unit of the second subset of the units.

In this way, it is possible to estimate the effect that an energy application in one area of the biological system has on other areas of the biological system. The determining of the simulated activation currents of the units of the second subset of units can also be performed in a time-dependent manner, thereby estimating the effects over time by determining a time-series of simulated activation currents.

It is furthermore possible to estimate the effect of energy applications in one area of the biological system on different scale (e.g. smaller or larger) structures than the areas which were mapped to individual units during the tuning method by using a scaling technique which is also referred to as multi-scale modelling.

To achieve such multi-scale modelling, the method for determining simulated activation currents may further include obtaining a secondary, i.e. different, larger or finer scale, biological network model, which comprises secondary units and respective connections of said secondary units. A set of secondary units may, for example, correspond to an area of the biological system which was previously mapped to a single unit in the biological network model. The connections of the secondary units are either known or estimated based on the measured data or based on additionally obtained measurement, for example imaging, data. For each of the secondary units, a secondary activity model also needs to be obtained or defined.

Simulated activation currents of the secondary units can then be determined based on the simulated input currents applied to the first set of units (or any set of units of the biological network model) and, furthermore, based on the connectivity information of the secondary units and, optionally, also based on input currents applied to some of the secondary units.

In the methods described above, the activity models of the individual units and subunits are usually initialized based on predetermined coupled differential equations which, in the case of brain network modelling are based on spiking neurons known from artificial neural networks, see [Reference 1].

Each connection of the brain network model furthermore has an associated weight to model a connection strength. While some of these weights may be fixed and pre-determined based on the used brain atlas or biological constants, at least some weights are adaptable in order to tune the brain network model to mimic the behaviour of the mammalian brain from which the measurement data was obtained.

Large Scale Brain Network Model

In the following, a specific model implementation and computer-based tuning method of a large scale brain network model is described. A large scale brain network model is a brain network model as described above with a multitude of units, corresponding to a multitude of measurement points or areas of the brain to be modelled. For example, a large scale brain network model may be based on the brain atlas of [Reference 2], which uses 379 brain areas for measurement, so that the corresponding brain network model then may use 379 units.

In general, the brain network model described below uses a natural number $N$ of units, based on a corresponding number of brain areas which were used during the obtaining of the measurement data. Indices i,j refer to natural numbers with $0 \leq i, j < N$. For example, $N = 379$ as discussed above.

A brain atlas, furthermore comprises connectivity information of the brain areas. This connectivity information is represented in the brain network model below by a connectome matrix $C$ of size $N$x$N$, which provides, for each $C_{ij}$, a connection strength from unit $i$ to unit $j$. In an implementation, the connectome matrix may be symmetric and/or $C_{ij} \in$

{0,1}$\forall 0 \leq i,j < N$ indicating either an existence or absence of connection between units $i$ and $j$. However, a more general implementation which allows for a non-symmetric matrix $C$ with generalized entries (for example entries that are from the interval [0,1)) is also possible.

Optionally, the matrix $C$ may be tunable, but in the example implementation provided below this is not the case, but $C$ may, however, be indirectly tunable as the entries of $C$ appear as multiplicative factors of tunable parameters.

Subscripts or superscripts E and I are used to distinguish parameters and/or variables of excitatory and inhibitory subunits, weights etc.

The large-scale brain network model discussed below simulates brain activity based on the network interaction of unit, also referred to as population models, that represent brain areas. Each brain area is thereby simulated by coupled excitatory and inhibitory subunits based on the dynamical mean field model, which was derived from a detailed spiking neuronal network model [Reference 1]. Units of the model are connected by connectome matrix $C$ which is based on structural connectomes estimated from dwMRI data via fiber tractography, see [Reference 3].

In this patent application, the model was extended using two new parameters $w_{ij}^{LRE}$ and $w_{ij}^{FFI}$ that allow the balancing of long-range excitatory and feedforward inhibitory synaptic currents. The model equations read as follows.

$$I_i^E = W_E I_0 + w_+ J_{NMDA} S_i^E + J_{NMDA} \sum_j w_{ij}^{LRE} C_{ij} S_j^E - J_i S_i^I \qquad (1)$$

$$I_i^I = W_I I_0 + J_{NMDA} S_i^E + J_{NMDA} \sum_j w_{ij}^{FFI} C_{ij} S_j^E - S_i^I \qquad (2)$$

$$r_i^E = \frac{a_E I_i^E - b_E}{1 - exp(-d_E(a_E I_i^E - b_E))} \qquad (3)$$

$$r_i^I = \frac{a_I I_i^I - b_I}{1 - exp(-d_I(a_I I_i^I - b_I))} \qquad (4)$$

$$\frac{dS_i^E(t)}{dt} = -\frac{S_i^E}{\tau_E} + (1 - S_i^E)\gamma_E r_i^E + \sigma v_i(t) \qquad (5)$$

$$\frac{dS_i^I(t)}{dt} = -\frac{S_i^I}{\tau_I} + \gamma_I r_i^I + \sigma v_i(t) \qquad (6)$$

In the formulas above, $r_i^{(E,I)}$ denotes the unit firing rate of the excitatory ($E$) and inhibitory ($I$) population of brain area $i$. $S_i^{(E,I)}$ identifies the average excitatory, respectively inhibitory, synaptic gating activity of each brain area and respective unit of the model. The sum of all input currents to each unit are identified by $I_i^{(E,I)}$ (units nA). $W_{(E,I)}I_0$ are the overall effective external input currents to excitatory, respectively inhibitory, subunits, and $w_+$ the local excitatory recurrence. $J_{NMDA}$ and $J_i$ are parameters that quantify the strengths of excitatory synaptic coupling and local feedback inhibitory synaptic coupling, respectively.

The summations in equations (1) and (2) are over all indices j of the set of units or, alternatively, only units j with connections to unit i (i.e. $C_{ij} \neq 0$) are taken into account.

Feedback inhibition control using inhibitory synaptic plasticity modulates $J_i$ of each region such that the long-term average firing rate $r_i^E$ of the corresponding excitatory population is roughly 4 Hz. Parameters $w_{ij}^{LRE}$ and $w_{ij}^{FFI}$ are matrices with the same dimensions as the structural connectome $C$ (i.e. $N$x$N$ for N units) that describe the strengths of long-range excitation and feedforward inhibition, respectively.

Equations 3 and 4 are sigmoidal functions that convert input currents into firing rates. $\tau_{(E,I)}$ and $\gamma_{(E,I)}$ specify the time scales and rate of saturation of excitatory and inhibitory synaptic activity, respectively. $v_i(t)$ is noise drawn from the standard normal distribution.

Table 1 lists all state variables used in the exemplary implementation of the brain network model. Said state variables are used in the coupled differential equations above to determined simulated activity levels (in the form of currents and firing rates) of each unit. For more details regarding these state variable, please see [Reference 1]. The values of the state variables form the basis of the tuning method discussed in this patent application, as the tuning is performed based on comparisons between the simulated activity levels expressed by the state variables and the measured activity of the measurement data.

Table 1: State variables

| Quantity | Value | Description |
|---|---|---|
| $r_i^{(E,I)}$ | | Population firing rate of the excitatory (E) or inhibitory (I) population in brain area i |
| $S_i^{(E,I)}$ | | NMDA (E) and GABA (I) synaptic gating |
| $I_i^{(E,I)}$ | | Sum of input currents |
| $v_i$ | | Noise sampled from the standard normal distribution |

Table 2 below lists preset parameters of the brain network model. In the exemplary implementation, the results of which are further discussed below, these parameters are set based on experimental observations and measured and or empirical data.

Table 2: Preset parameters

| Quantity | Value | Description |
|---|---|---|
| $I_0$ | 0.382 nA | Overall effective external input |
| $W_{(E,I)}$ | 1 (E); 0.7 (I) | Modulation of $I_0$ for excitatory, respectively, inhibitory population |
| $w_+$ | 1.4 | Local excitatory recurrence |
| $J_{NMDA}$ | 0.15 nA | Excitatory synaptic coupling |
| $C_{ij}$ | Obtained by dwMRI tractography | Structural connectivity matrix |
| $a_E, b_E, d_E$ | 310 (1/nC), 125 (Hz), 0.16 (s) | Parameters of excitatory population's frequency-current (f-I) function |
| $a_I, b_I, d_I$ | 615 (1/nC), 177 (Hz), 0.087 (s) | Parameters of inhibitory population's f-I function |
| $\gamma_E, \gamma_I$ | $6.41 \times 10^{-4}$, $1.0 \times 10^{-3}$ | Rate of saturation |
| $\tau_E, \tau_I$ | 100 ms, 10 ms | Time scales of synaptic activity |
| $\sigma$ | 0.01 | Noise scaling |

Table 3 below lists the tunable parameters. These parameters include the long-range excitation and feed forward inhibitions weights as well as the local inhibitory weights, which are referred to as feedback inhibitory synaptic coupling variables below. The feed forward inhibition and long range excitation matrices $w^{LRE}$ and $w^{FFI}$ are tuned by the tuning method described below, which is based on the functional connectivity measurements (hence referred to as FC fitting or FC tuning) while the local inhibitory weights are determined based on a so-called feedback inhibition control method, or FIC-method, which is also described below. Both tuning methods, i.e. FC-tuning and FIC can be performed together or intermittently, as also discussed below.

Table 3: Tunable Parameters

| Quantity | Value | Description |
|---|---|---|
| $w_{ij}^{LRE}$ | Obtained by FC fitting | Long-range excitation |
| $w_{ij}^{FFI}$ | Obtained by FC fitting | Feedforward inhibition |
| $J_i$ | Obtained by FIC | Feedback inhibitory synaptic coupling |

The functional connectivity based tuning method is based on the observation that the correlation between the simulated fMRI time series from two different units can be modulated by the relative strengths of long-range excitation versus feedforward inhibition.

Said correlation between the simulated fMRI time series of different units is expressed by the simulated functional connectivity, which expresses the pair-wise correlation between the simulated fMRI time series of the units of the brain network model. To obtain simulated fMRI time series, the simulated firing rates (equations 3 and 4) are used to calculate the simulated neural or synaptic activities (equations 5 and 6). The calculated simulated neural or synaptic activity is then input into the Balloon-Windkessel model, which simulates the coupling between neural or synaptic activity and blood oxygenation levels and changes, thereby determining the simulated fMRI measurements. For details regarding the Balloon Windkessel model including specific model equations that may be used in the described tuning method see [Reference 1].

This ratio can be adjusted in the described model by the parameters $w_{ij}^{LRE}$ and $w_{ij}^{FFI}$, which are multiplicative factors that re-scale the structural connectivity weights $C_{ij}$, between each pair of connected units i and j. $w_{ij}^{LRE}$ modulates the amount of excitation conveyed via long-range connections to distant excitatory subunits, or long-range excitation, while $w_{ij}^{FFI}$ modulates the amount of excitation provided via long-range connections to distant inhibitory subunits, and their resulting feedforward inhibitory effect on the accompanying excitatory population, or feedforward inhibition.

The goal of the tuning method is to fit weights $w_{ij}^{LRE}$ and $w_{ij}^{FFI}$ such that the simulated functional connectivity computed from simulated fMRI time series based on the coupled differential equations (1) to (6) above matches a target functional connectivity, determined based on fMRI scans of the brain of a patient, as closely as possible. The goal is that the difference between each entry $\rho_{ij}^{trg}$ of the target functional connectivity matrix $\rho^{trg}$ and $\rho_{ij}^{sim}$ of the simulated functional connectivity matrix $\rho^{sim}$ should be as small as possible. The basic idea of the tuning method is to increase $w_{ij}^{LRE}$ and to decrease $w_{ij}^{FFI}$ if $\rho_{ij}^{trg} > \rho_{ij}^{sim}$ and vice versa, to decrease $w_{ij}^{LRE}$ and to increase $w_{ij}^{FFI}$ if $\rho_{ij}^{trg} < \rho_{ij}^{sim}$. In pseudocode the algorithm can be written as follows.

**Method FC_tuning($\rho^{trg}, \eta$, M)**

**Input** $\rho^{trg}$ : (n x n) target FC matrix
$\eta$:  scalar learning rate
M:  brain network model

**Returns**      $w^{LRE}$ (n x n) matrix of long-range excitation weights,
$w^{FFI}$ (n x n) matrix of feedforward inhibition weights

**for** fmri_time_step = 1 **to** simulation_length **do**
simulate one fMRI time step using M
compute simulated FC $\rho^{sim}$
**for** i = 1 **to** n **do**
rmse_i = root-mean-square deviation between matrix rows i in $\rho^{trg}$ and $\rho^{sim}$:
**for** j = 1 to number of connections of node i **do**
diff_FC = $\rho_{ij}^{trg}$ - $\rho_{ij}^{sim}$
$w_{ij}^{LRE}$ = $w_{ij}^{LRE}$ + $\eta$ x diff_FC x rmse_i
$w_{ij}^{FFI}$ = $w_{ij}^{FFI}$ - $\eta$ x diff_FC x rmse_i
**if** $w_{ij}^{LRE} \leq 0$ **do** $w_{ij}^{LRE} = 0$
**if** $w_{ij}^{FFI} \leq 0$ **do** $w_{ij}^{FFI} = 0$
**return** $w^{LRE}$, $w^{FFI}$

The algorithm iterates over all connections (*i,j*) of the brain network model and computes the difference between target and simulated functional connectivity for each connection. This difference is rescaled by the learning rate $\eta$, which is gradually decreased over the course of the tuning. Furthermore, the difference is re-scaled by the root-mean-square deviation (RMSE) between the correlation coefficient values of unit i with all remaining units (i.e. the RMSE between rows *i* of matrices $\rho^{trg}$ and $\rho^{sim}$), which can be compared to the temperature parameter in a simulated annealing heuristic. The factor has the purpose to decrease the change in $w_{ij}^{LRE}$ and $w_{ij}^{FFI}$ as the fit of the row-wise functional connectivity increases and we approach an optimum. Furthermore, the factor differentially weights the changes in $w_{ij}^{LRE} (w_{ij}^{FFI})$ and $w_{ji}^{LRE} (w_{ji}^{LRE})$ with the purpose that the unit (either *i* or *j*) that has a better fit at the current tuning iteration is changed less than the other one, since the change of connection strengths between one unit pair has an effect on the functional connectivity between all other unit pairs. By decreasing the step size for the better-fitting unit, it is ensured that respective parameters stay closer to the local optimum. This heuristic is used as an online parameter tuning rule, which means that parameters are updated after each new BOLD fMRI time step is computed. Thus, initially the parameter space is sampled with large steps (large learning rate) using simulated functional connectivity that is based on a short time window. In successive tuning stages, $\eta$ is decreased and the window size for simulated functional connectivity computation is increased.

At the beginning of the FC_tuning method, the tuning variables, i.e. the feed forward inhibition weights $w_{ij}^{FFI}$ and long range excitation weights $w_{ij}^{LRE}$ are initialized. Experiments have shown that initializing the weights to any values between 0 and 10 produces good convergent tuning results. However, other initial values may also work well depending on the scaling factors and learning rates which are used in a specific implementation.

Feedback inhibition control

The firing rate of the simulated brain units (Eqs. 3, 4) depends on simulated synaptic input currents (Eqs. 1, 2), which are, to a large degree, determined by the structural connectome *C,* that is, large-scale inputs, and associated parameters ( $w_{ij}^{LRE}$ and $w_{ij}^{FFI}$ ). To compensate for excess or lack of excitation, which would result in implausible firing rates, a local regulation mechanism, called feedback inhibition control (FIC), may be used. To implement FIC a learning rule for inhibitory synaptic plasticity is used to balance excitation and inhibition in sensory pathways and memory networks. The learning rule modulated all connection strengths from inhibitory to local excitatory populations once every 720 ms (corresponding to 1 fMRI repetition time) to achieve a target average firing rate of 4 Hz in excitatory populations. The learning time window can be adapted based on the used fMRI machine. Also, note that measurement is usually taken for a respective time window, i.e. activity over a certain period of time is measured. The learning rule can be summarized as

$$\Delta w = \eta(pre \times post - \rho_0 \times pre) \qquad (7)$$

where $\Delta w$ denotes the change in simulated synaptic strength, *pre* and *post* are the simulated pre- and postsynaptic firing rates, $\eta$ = 0.001 is the learning rate and $\rho_0$ = 4.0 [*Hz*] is the target firing rate for the simulated postsynaptic excitatory population. If the simulated postsynaptic firing rate *post* is larger than the target firing rate $\rho_0$, the learning rule (7) increases the inhibitory weight *w,* to decrease the simulated postsynaptic firing rate. Conversely, if the simulated postsynaptic firing rate is lower than the target firing rate, the learning rule decreases the inhibitory weight. The change of the inhibitory weight is modulated by the simulated presynaptic firing rate *pre*: if the simulated presynaptic firing is large, then a higher weight change is needed to get the desired effect than when presynaptic firing is low. The learning rate $\eta$ was preset and may be optimized by experimental results.

The tuning method is highly complex and can only be executed in an efficient (and usable) manner by a computer system. Even using a computer system, the tuning method may take several hours to several days, depending on the number of units.

Determining simulated currents of a tuned brain network model

A tuned brain network model, for example the brain network model which has been tuned based on an fMRI scan of a patient's brain according to the tuning method discussed above, can then be used to simulate and, thereby predict, activities in the form of firing rates and input currents of the brain areas. In particular, it is possible to estimate a (local and global) effect of stimulation on an area of the patient's brain by inputting the stimulation activities to the respective unit of the brain network model and then calculate the resulting firing rates and input currents of other units, thereby predicting activities of other brain areas of the patient.

Please note that the simulation method is also highly complex and can only be executed in an efficient manner using a computer system, in particular as several different stimulation currents and stimulation locations are usually considered.

Using this simulation it is possible to accurately estimate the effect of stimulation of a location within a patient's brain. This makes it possible to identify locations for which stimulation causes the greatest benefit to the patient, thereby reducing the need for trial and error during a subsequent surgical intervention. The method therefore reduces the risk of injury to the patient and also reduces the necessary resources, where resources refer to time as well as material resources such as surgical supplies and laboratory animals, which may otherwise be necessary to estimate the effect of stimulation on other brain areas.

To obtain the simulated activation currents, the equations (1) - (6) can be used, however with fixed or pre-obtained long range excitation and feed forward inhibition weights. Additionally, local inhibitory weights may also be fixed Then, the state variables may be initialized with values that correspond to a stimulation energy applied in a given area of the brain.

Based on a tuned brain network model or a certain number of units, it is also possible to adapt the scale of selected regions of the brain network model, thereby enabling simulation on a different scale than the scale which was used during the tuning method. In particular, it is possible to define a multi-scale brain network model, as can be seen below and to then simulate and predict activities on a different, i.e. finer or larger scale. In such a multiscale brain network model, some areas may be rescaled, while other areas are not rescaled or are scaled differently.

The equations below provide a specific implementation of a multi-scale brain network model, where the original brain network model, BNM, is used to determine activation currents for the "target" model.

Below, equation (8) corresponds to a summation of the input currents of the fine-scale "target" model, which uses a connectivity matrix $C_{ij}^{target}$. Equation (8), thereby, corresponds to the input current Equations of the large scale brain network model (also referred to as "source", similar to equations (1) and (2) described above. The coupling equation (12) described the inter-relation between the large scale model "source" and the fine scale model "target". In particular, parameters $u_{target}$, $l_{target}$ relate to the "upper" and "lower" limit of the target currents, while $u_{source,i}$, $l_{source,i}$ relate to the corresponding "upper" and "lower" limits of the source currents of unit, or region, $i$. Thus, a source current of range $[u_{source,i}, l_{source,i}]$ is rescaled to a target current of range $[u_{target}, l_{target}]$.

The values $\eta_i(t)$ are random values samples from a normal distribution of mean 0.0 and variance 1.0.

$$I_i^{target} = \sum_j C_{ij}^{target} S_j^{target} + I_0^{target} + I_{app,i}^{target} + I_{BNM,i}^{target} \qquad (8)$$

$$r^{target}(I^{target}) = \frac{aI^{target} - b}{1 - exp(-c(aI^{target} - b))} \qquad (9)$$

$$\frac{dS_i^{target}}{dt} = -\frac{S_i^{target}}{\tau} + \gamma(1 - S_i^{target})r^{target}(I_i^{target}) \qquad (10)$$

$$\tau_{AMPA}\frac{dI_{noise,i}^{target}(t)}{dt} = -I_{noise,i}^{target}(t) + \eta_i(t)\sqrt{\tau_{AMPA}\sigma_{noise}^2} \qquad (11)$$

$$I_{BNM,i}^{target} = (u_{target} - l_{target})[(w_+J_{NMDA}S_i^E + J_{NMDA}\sum_j w_{ij}^{LRE} C_{ij}S_j^E$$

$$- J_iS_i^I) - l_{source,i}] / (u_{source,i} - l_{source,i}) + l_{target}$$

$$+ I_{noise,i}^{target}(t) \qquad (12)$$

Definitions of the variables and parameters are provided below in addition to the parameters and variables which were already defined above in Table 1, 2 and 3.

Table 4: Parameters and variables for multi-scale modelling

| Quantity | Value | Description |
|---|---|---|
| *State variables* | | |
| $r^{target}$ | | Population firing rates |
| $S_i^{target}$ | | NMDA synaptic gating of *target* population i |
| $I_i^{target}$ | | Sum of input currents of *target* population i |

(continued)

| Quantity | Value | Description |
|---|---|---|
| *State variables* | | |
| $I_{noise,i}^{target}$ | | Noise current of *target* popula-tion i |
| $I_{app,i}^{target}$ | | Applied current to simulate the effect of external stimuli |
| *Parameters* | | |
| $I_0^{target}$ | 0.334 nA | Overall effective external input of *target* circuit populations |
| $C_{i,j}^{target}$ | Pre-determined or supplied | Connectivity matrix of *target* cir-cuit populations |
| *a b c* | 270 (1/nC), 108 (Hz), 0.154 (s) | Parameters of frequency-current (f-I) function |
| $\gamma$ | $6.41 \times 10^{-4}$ | Kinetic parameters |
| $\tau$ | 60 ms | Time constant of NMDA synaptic activity |
| $\tau_{AMPA}$ | 2 ms | Time constant of AMPA synaptic activity |
| $\sigma_{noise}$ | 0.009 nA | Noise strength |

Using the examples and implementations described above, it is possible to accurately tune a brain network model based on non-invasive medical imaging data of an individual brain. Furthermore, it is, then, possible to estimate the effect of stimulations currents on the individual brain based on a tuned model and to even adapt the scale of stimulation for additional estimation of fine scale or large scale estimation of the effect of stimulation.

The detailed examples and implementations provided above only relate to one specific example for implementing the inventive method and system. Other examples and implementations which fall within the scope of the appended claims, including different variables, parameters and formulas, are also encompassed by the scope of protection.

## Claims

1. Computer-implemented method for tuning a biological network model (40) based on measured data of a biological system, the method comprising the steps:

   - Obtaining (Step 501, 601) the measured data of the biological system, the measured data comprising structural data of the biological system and activity data of the biological system;
   - Obtaining (Step 602) a measured structural connectivity of the biological system, wherein the measured structural connectivity is determined based on the structural data of the biological system;
   - Obtaining (Step 603) a measured functional synchronization of the biological system, wherein the measured functional synchronization is determined based on the measured activity data;
   - Initializing (Step 502) the biological network model based on the measured data, wherein the biological network model comprises

     ∘ a set of units (41, 42, 43) and connectivity information of said units (41, 42, 43), determined based on the measured structural connectivity, each unit (41, 42, 43) comprising an activity model, an inhibitory subunit (41-I, 42-I, 43-I) and an excitatory subunit (41-E, 42-E, 43-E), wherein the excitatory subunit (41-E, 42-E, 43-E) of at least some units (41, 42, 43) is functionally coupled to the inhibitory subunit (41-I, 42-I, 43-I) of the same unit (41, 42, 43) and vice versa, wherein the activity model defines a time-dependent simulated activity of the respective unit (41, 42, 43);
     ∘ a set of long range excitation weights, each of which represents a coupling strength from the excitatory subunit (41-E, 42-E, 43-E) of one unit (41, 42, 43) to the excitatory subunit (41-E, 42-E, 43-E) of another unit (41, 42, 43);
◦ a set of feed forward inhibition weights, each of which represents a coupling strength from the excitatory subunit (41-E, 42-E, 43-E) of one unit (41, 42, 43) to the inhibitory subunit (41-I, 42-I, 43-I) of another unit (41, 42, 43);

- tuning the biological network model (40) by repeatedly performing the following steps until a stopping condition is met:

◦ determining a simulated synchronization of a selected subset of the units (41, 42, 43) based on the simulated activity of said selected subset of the units (41, 42, 43), wherein the simulated activity of each unit (41, 42, 43) is determined based on the activity model of said unit (41, 42, 43);
◦ determining a deviation between the simulated synchronization and a corresponding subset of the measured functional synchronization of the biological system;
◦ adapting a proper, non-empty subset of the long range excitation weights and/or a proper non empty subset of the feed forward inhibition weights based on the deviation.

2. The method of claim 1, wherein the adapting the subset of the long range excitation weights and/or the subset of the feed forward inhibition weights based on the deviation comprises that, for a first unit (41, 42, 43) and a second unit (41, 42, 43), the long range excitation weight from the first unit (41, 42, 43) to the second unit (41, 42, 43) and the feed forward inhibition weight from the first unit (41, 42, 43) to the second unit (41, 42, 43) are adapted interdependently

3. The method of claim 2, wherein the interdependent adaptation of the long range excitation weight and the feed forward inhibition weight from the first unit (41, 42, 43) to the second unit (41, 42, 43) comprises increasing one of said two weights while decreasing the other one of said two weights.

4. The method of any of the preceding claims, wherein the adapting the subset of the long range excitation weights and/or the subset of the feed forward inhibition weights based on the deviation comprises scaling the deviation according to a fixed and/or dynamic scaling factor to determine a scaled deviation, and adapting the subset of the long range excitation weights and/or the subset of the feed forward inhibition weights based on the scaled deviation.

5. The method of any one of the preceding claims wherein the simulated synchronization of the subset of the simulated units is determined based at least in part on the temporal correlation of the activities of said subset of the simulated units (41, 42, 43).

6. The method of any one of the preceding claims, wherein the biological system is a mammalian brain and the biological network model (40) is an individual brain network model and/or wherein the measured functional synchronization is a functional connectivity determined based on the measured data (201-a, 201-b, 202) of the biological system.

7. The method of any one of the preceding claims, wherein the activity model defines a temporal dependency between a current excitatory activity of a specific unit (41, 42, 43) and previous excitatory and/or inhibitory activities of connected units (41, 42, 43), which are connected to the specific simulated unit (41, 42, 43) according to the structural connectivity information, and
wherein the activity model defines a temporal dependency between a current inhibitory activity of the specific unit (41, 42, 43) and previous excitatory and/ or inhibitory activities of the connected units (41, 42, 43).

8. The method of claim 7, wherein

- the specific unit's current excitatory activity is determined based at least in part on a weighted sum over a set of previous simulated excitatory activities of the connected units (41, 42, 43), which are weighted by the respective long range excitation weights; and
- the specific unit's current inhibitory activity is determined based at least in part on a weighted sum over a set of previous simulated excitatory activities of the connected units (41, 42, 43), which are weighted by the respective feed forward inhibition weights.

9. The method of any one of the preceding claims, wherein, for at least one unit (41, 42, 43), the excitatory subunit and the inhibitory subunit of said unit are coupled by a local excitatory weight representing a coupling strength from the excitatory subunit (41-E, 42-E, 43-E) to the inhibitory subunit (41-I, 42-I, 43-I), and a local inhibitory weight representing a coupling strength from the inhibitory subunit to the excitatory subunit.

10. The method of claim 9, wherein the method furthermore comprises a step of adapting the local inhibitory weight of the unit (41, 42, 43) based on a difference between said unit's activity and a target activity, wherein the target activity may preferably be a target excitatory firing rate between 2 Hz and 8 Hz.

11. The method of any of the preceding claims, wherein the biological network model comprises a number N of units (41, 42, 43), and the long range excitation weights are represented by a long range excitation matrix of size NxN, the feedforward inhibition weights are represented by a feed forward inhibition matrix of size NxN, and/or the local inhibitory weights are represented by a local inhibitory vector of size N.

12. A computer implemented method for determining simulated activation currents of a biological network model, the method comprising

- Obtaining (Step 901) the biological network model, the biological network model comprising

    ○ a set of units and connectivity information of said units, each unit comprising an activity model, an inhibitory subunit and an excitatory subunit, wherein the excitatory subunit of a unit is functionally coupled to the inhibitory subunit of the same unit and vice versa, wherein the activity model defines a time-dependent simulated activity of the respective unit;
    ○ a set of long range excitation weights, each of which represents a coupling strength from the excitatory subunit of one unit to the excitatory subunit of another unit;
    ○ a set of feed forward inhibition weights, each of which represents a coupling strength from the excitatory subunit of one unit to the inhibitory subunit of another unit;

- applying (Step 902) a simulated input current to each unit of a first subset of the units;
- determining (Step 903) the simulated activation currents of each unit of a second subset of the units, wherein the simulated activation currents of a unit of the second subset of the units is determined based on the simulated input currents applied to the units of the first subset of the units, and further based on the activity model of said unit of the second subset of the units.

13. The method of claim 12, wherein the biological network model was obtained by the tuning method according to any one of claims 1 to 11.

14. The method of claim 12 or 13, the method further comprising:

- obtaining a secondary biological network model comprising a set of secondary units and secondary connectivity information of said secondary units, wherein the set of secondary units corresponds to a different scale model of a specific unit of the set of units of the biological network model, each secondary unit comprising a secondary activity model; and
- determining simulated activation currents of at least some of the secondary units, wherein the simulated activation currents of the at least some secondary units are determined based on simulated input currents applied to the first subset of the units and/or based on input currents applied to at least some of the secondary units.

15. A data processing apparatus comprising means for carrying out the method steps of any one of the preceding claims.

FIG. 1

FIG. 2

FIG. 3

FIG. 4

```
┌─────────────────┐
│       501       │
└─────────────────┘
         │
         ▼
┌─────────────────┐
│       502       │
└─────────────────┘
         │
         ▼
┌─────────────────┐
│       503       │
└─────────────────┘
```

**FIG. 5**

501

```
┌───────────────────────────┐
│   ┌───────────────────┐    │
│   │        601        │    │
│   └───────────────────┘    │
│            │               │
│            ▼               │
│   ┌───────────────────┐    │
│   │        602        │    │
│   └───────────────────┘    │
│            │               │
│            ▼               │
│   ┌───────────────────┐    │
│   │        603        │    │
│   └───────────────────┘    │
└───────────────────────────┘
```

**FIG. 6**

502

701

702

703

704

**FIG. 7**

503

801

803

804

805

805b

805a

**FIG. 8A**

FIG. 8B

FIG. 9

## EUROPEAN SEARCH REPORT

| | Application Number |
|---|---|
| | **EP 23 16 6587** |

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/206051 A1 (MCLNTOSH ANTHONY RANDAL [CA] ET AL) 23 July 2015 (2015-07-23) * paragraphs 31, 2, 13, 47, 9, 28, 30, 32, 107, 109, 52, 75, 106, 108, 133 * ----- | 1-15 | INV. G16H50/50 |

| | |
|---|---|
| | TECHNICAL FIELDS SEARCHED (IPC) |
| | G16H |

The present search report has been drawn up for all claims

| Place of search | Date of completion of the search | Examiner |
|---|---|---|
| Munich | 22 September 2023 | Wittke, Claudia |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
......................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 23 16 6587

This annex lists the patent family members relating to the patent documents cited in the above-mentioned European search report.
The members are as contained in the European Patent Office EDP file on
The European Patent Office is in no way liable for these particulars which are merely given for the purpose of information.

22-09-2023

| Patent document cited in search report | | Publication date | Patent family member(s) | | Publication date |
|---|---|---|---|---|---|
| US 2015206051 A1 | | 23-07-2015 | CA | 2880758 A1 | 06-02-2014 |
| | | | EP | 2880600 A2 | 10-06-2015 |
| | | | US | 2015206051 A1 | 23-07-2015 |
| | | | US | 2019251450 A1 | 15-08-2019 |
| | | | WO | 2014020428 A2 | 06-02-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DECO et al.** How Local Excitation/Inhibition Ratio Impacts the Whole Brain Dynamics. *The Journal of Neuroscience,* vol. 34 (23), 7886-7898
- **GLASSER, MATTHEW F. et al.** A multi-modal parcellation of human cerebral cortex. *Nature,* 2016, vol. 536 (7615), 171-178
- **SPORNS, OLAF ; GIULIO TONONI ; ROLF KÖTTER.** The human connectome: a structural description of the human brain. *PLoS computational biology,* 2005, vol. 1 (4), e42
- **RAICHLE, MARCUS E. et al.** A default mode of brain function. *Proceedings of the national academy of sciences,* 2001, vol. 98 (2), 676-682